# EUROPEAN PATENT SPECIFICATION

(11) **EP 3 570 868 B1**
(45) Date of publication and mention of the grant of the patent: **20.05.2020**
(21) Application number: 17704656.2
(22) Date of filing: 20.01.2017
(51) Int. Cl.: A61K 38/17, C07K 7/08, C07K 14/435

(54) **SYNTHETIC ANTIMICROBIAL PEPTIDES AND THEIR APPLICATIONS FOR THE TREATMENT AND PREVENTION OF MUSCULOSKELETAL INFECTIONS**
SYNTHETISCHE ANTIMIKROBIELLEN PEPTIDE UND DEREN VERWENDUNG ZUR BEHANDLUNG UND VORBEUGUNG VON MUSKOLOSKELETALEN INFEKTIONEN
PEPTIDES ANTIMICROBIELS SYNTHÉTIQUES ET LEUR USAGE DANS LE TRAITEMENT ET PRÉVENTION DES INFECTIONS MUSCULOSQUELETTIQUES

(43) Date of publication of application: 27.11.2019
(73) Proprietor: Ustav Organicke Chemie a Biochemie AV CR, v.v.i., 16610 Praha 6 (CZ); Fakultni Nemocnice V Motole, 15006 Praha 5 (CZ)
(72) Inventor: CEROVSKY, Vaclav, 25263 Roztoky (CZ); MELICHERCIK, Pavel, 10200 Praha 10 (CZ); NESUTA, Ondrej, 74707 Opava (CZ)
(74) Representative: Hartvichova, Katerina
(86) International application number: PCT/CZ2017/050001
(87) International publication number: WO 2018/133887

(56) References cited:
- L MONINCOVA ET AL.: "Novel antimcrobial peptides from the venom of the eusocial bee Halictus sexcinctus (hymonoptera: Halictidae) and their analogs", AMINO ACIDS., vol. 39, 2010, pages 763-775, XP002771460, SPRINGER VERLAG. ISSN: 0939-4451
- S M DE FREITAS LIMA ET AL.: "Antimicrobial peptide-based treatment for endodontic infections - Biotechnological innovations in endodontics", BIOTECHNOLOGY ADVANCES., vol. 33, no. 1, 2015, pages 203-213, XP002771461, ELSEVIER PUBLISHING, BARKING. ISSN: 0734-9750
- K DE BRUCKER ET AL.: "Derivatives of the mouse cathelicidin-related antimicrobial peptide (CRAMP) inhibit fungal and bacterial biofilm formation", ANTIMICROBIAL AGENTS AND CHEMOTHERAPY, vol. 58, no. 9, September 2014 (2014-09), pages 5395-5404, XP002771462, American Society for Microbiology ISSN: 0066-4804

## Description

### Field of Art

The invention involves describes peptides of formulae I-XIII and their selected enantiomers as well as their use to treat musculoskeletal infections in orthopaedics and traumatology, including applications on implants used in orthopaedics, especially when mixed with the local carrier.

### Background Art

Musculoskeletal infections still belong to frequent postoperative complications in orthopaedics and traumatology. Despite improving prevention, surgery techniques, postoperative care and better access to antibiotics, they bring a serious danger due to their typical progression with relapses and frequent development of resistance. In medical practice, these include primarily osteomyelitis and infections associated with implants used in orthopaedics and traumatology.

Treatment of osteomyelitis (bone infection) must be comprehensive and long-term. It is based on antimicrobial therapy (currently using antibiotics), combined with surgical treatment and adjuvant therapy. The key problem is the solution of "dead space" in bone after debridement, which involves removing sequesters, eradication of infection and solving problems with the formation of bacterial biofilms. One of the ways to increase the therapeutic potential and to fill in the cavity of the bone affected with infection is the use of local carriers mixed with antibiotics [1-4]. Infection of implants used in orthopaedics, such as joint replacements, materials used for their fixation and osteosynthesis materials represent another serious problem. In these cases, the infection is associated with the formation of microbial biofilms on the implant surfaces [5]. Microbial biofilms are for example very easily formed on an implant prepared from bone cement, which is based on polymethyl methacrylate [6]. Even in this case, the solution is to mix the cement with antibiotics just prior to use, or to use cement, whose powder component contains antibiotics from the producer.

Local carriers for the antibiotics used in orthopaedics can be divided into synthetic and natural polymers, ceramics, composites, and bone grafts. In clinical practice, calcium phosphate, calcium sulphate, collagen, bone grafts, hydroxyapatite and bone cement (polymethyl methacrylate) are the most commonly used. Local carriers with antibiotics can be used both for treatment and prevention of osteomyelitis by filling the "dead" area of the infected bone, but also for the prevention of microbial biofilms on the surfaces of implants. Antimicrobial treatment of musculoskeletal infections with local carriers with antibiotics, compared to systemic application of antibiotics, has the advantage of achieving concentrations of antimicrobial agents that are far beyond their minimum inhibitory concentration (MIC). Antibiotics released from local carriers thus act also in avascular zones and without increasing systemic toxicity. Antibiotics slowly released from the bone cement prevents its surface colonization by microbes and subsequent formation of microbial biofilm.

The big problem in orthopaedics, however, is the resistance of some microbes to currently available antibiotics or antifungal agents. These are mainly methicillin resistant *Staphylococcus aureus* (MRSA), *Staphylococcus epidermidis, Streptococcus species* and *Pseudomonas aeruginosa.* Alarming is the occurrence of *Staphylococcus aureus* resistant to vancomycin, as this is often used as a last resort antibiotic. A very serious danger is caused by yeast infections of the *Candida* genus. These microbes colonize bone tissue or implants, and adhere to the surface with the consequent formation of biofilms. The infected bone tissue is difficult to treat, since microbes in the biofilm are many times more resistant to antimicrobials compared to planktonic microbes. In the case of biofilms formed on an implant, such as a joint replacement, the solution is to remove the implant from the body, mechanical removal of the biofilm, the necrotic tissue, and wear-induced granuloma, disinfection, local application of antimicrobial agents and treatment of the resulting "dead" space with several weeks of systemic administration of antimicrobial agents. Subsequent insertion of a new, usually a more complex implant may be made either at one time, or more frequent procedure - two-stage re-implantation using so-called spacers containing antibiotics or antifungal agents [7,8]. These procedures are always long, exhausting for the patient and very costly.

Very promising therapeutic method could be the use of antimicrobial peptides (AMPs) released from the local carriers to the site of bone infection, or their incorporation into implants and bone cements. Since the AMPs kill bacteria or yeasts with a totally different mechanism than traditional antibiotics and in doing so do not generate bacterial resistance [9], they have so far only been considered as a supplement to traditional antibiotics or their substitution [10-16]. Our newly discovered highly effective AMPs offer the use of preferred, synthetically available compounds for eradication of bone infections or preventing the formation of microbial biofilms on the implant surfaces, particularly after incorporation into local carriers.

Antimicrobial peptides have been identified in virtually the entire spectrum of animal and plant kingdom. For example, in the realm of insects, precisely these peptides are the main means of defence against microbes [17,18]. Taking into account the environment in which the insect lives, these must be very effective peptides. These peptides are able to rapidly kill bacteria and other microorganisms by a mechanism quite different from commonly used antibiotics. Although this mechanism is not yet fully understood, basically it involves disruption of the cell membrane of bacteria or yeast by formation of transmembrane ion channels or pores. Subsequent leakage of metabolites causes a collapse of the entire microbial structure [19-21]. Generally it is also known that antimicrobial peptides in bacteria do not cause resistance, which is known in the heretofore used conventional antibiotics.

De Brucker K. et al., Antimicrobial agents and chemotherapy, vol. 58, no. 9, 2014-09, pages 5395-5404, disclose a derivative of the mouse cathelicidin-related antimicrobial peptide that inhibit fungal and bacterial biofilm formation, having a putative application as an antibiofilm coating on medical devices and implants.

In our laboratory, we developed shorter antimicrobial peptides known as halictins, named after the natural peptide halictin II and consisting of 12 amino acids, having a strong antimicrobial activity against pathogenic bacteria and yeasts. It has now surprisingly been found that with targeted variation of their amino acid composition, or shortening of the chain by one amino acid at the N-terminus, a group of synthetic analogues can be obtained which, unlike the previously synthesized halictins, retain antimicrobial activity even after incorporation into local carriers or bone cement. From these carriers, they are then readily released into the infected bone tissue and eradicate the infection. At the same they time exhibit the ability to actively prevent the formation of microbial biofilm on the surface of the implant formed from polymethylmethacrylate bone cement, to which this antimicrobial peptide was added. These peptides have also extraordinary antimicrobial effect in bone tissue and reduced haemolytic activity, which allows their use in the presence of bone cells. It was also found that other changes, apparently very similar to the modifications performed in the sequence of these peptides, lead to analogues which don't exhibit such a pronounced effect in the infected bone tissue, and moreover, they have the undesirable haemolytic activity.

### Disclosure of the Invention

Subject matter of the invention involves antimicrobial peptides of the general formula
**a**-Lys-Trp-**b**-Lys-**c**-Leu-Lys-Lys-Ile-Leu-Lys-NH₂ (A)
where **a** is Gly, βAla or there isn't any aminoacid,
**b** is Met, Met(O), Leu, Val, or Lys,
**c** is Leu, Met or Val,
showing an antimicrobial effect in bone tissue,
and being selected from the group consisting of:
H-Lys-Trp-Met-Lys-Leu-Leu-Lys-Lys-Ile-Leu-Lys-NH₂ (I) (SEQ ID NO. 1),
*H-Lys-Trp-Met-Lys-Leu-Leu-Lys-Lys-Ile-Leu-Lys*-NH₂ (II) (SEQ ID NO. 2),
H-Gly-Lys-Trp-Met-Lys-Leu-Leu-Lys-Lys-Ile-Leu-Lys-NH₂ (III) (SEQ ID NO. 3),
H-Gly-*Lys-Trp-Met-Lys-Leu-Leu-Lys-Lys-Ile-Leu-Lys*-NH₂ (IV) (SEQ ID NO. 4),
H-βAla-Lys-Trp-Met-Lys-Leu-Leu-Lys-Lys-Ile-Leu-Lys-NH₂ (V) (SEQ ID NO. 5),
H-βAla-*Lys-Trp-Met-Lys-Leu-Leu-Lys-Lys-Ile-Leu-Lys*-NH₂ (VI) (SEQ ID NO. 6),
H-Gly-Lys-Trp-Leu-Lys-Leu-Leu-Lys-Lys-Ile-Leu-Lys-NH₂ (VII) (SEQ ID NO. 7),
*H-Gly-Lys-Trp-Leu-Lys-Leu-Leu-Lys-Lys-Ile-Leu-Lys*-NH₂ (VIIa) (SEQ ID NO. 7),
H-Gly-Lys-Trp-Val-Lys-Leu-Leu-Lys-Lys-Ile-Leu-Lys-NH₂ (VIII) (SEQ ID NO. 8),
H-Gly*-Lys-Trp-Val-Lys-Leu-Leu-Lys-Lys-Ile-Leu-Lys*-NH₂ (IX) (SEQ ID NO. 9),
H-Gly-Lys-Trp-Lys-Lys-Leu-Leu-Lys-Lys-Ile-Leu-Lys-NH₂ (X) (SEQ ID NO. 10),
*H-Gly-Lys-Trp-Lys-Lys-Leu-Leu-Lys-Lys-Ile-Leu-Lys*-NH₂ (Xa) (SEQ ID NO. 10),
H-Gly-Lys-Trp-Met-Lys-Met-Leu-Lys-Lys-Ile-Leu-Lys-NH₂ (XI) (SEQ ID NO. 11),
*H-Gly-Lys-Trp-Met-Lys-Met-Leu-Lys-Lys-Ile-Leu-Lys*-NH₂ (XIa) (SEQ ID NO. 11),
H-Gly-Lys-Trp-Met(O)-Lys-Leu-Leu-Lys-Lys-Ile-Leu-Lys-NH₂ (XII) (SEQ ID NO. 12),
*H-Gly-Lys-Trp-Met(O)-Lys-Leu-Leu-Lys-Lys-Ile-Leu-Lys*-NH₂ (XIIa) (SEQ ID NO. 12),
H-Gly-Lys-Trp-Met-Lys-Leu-*Leu*-Lys-Lys-Ile-Leu-Lys-NH₂ (XIII) (SEQ ID NO. 13),
*H-Gly-Lys-Trp-Met-Lys-Leu-*Leu*-Lys-Lys-Ile-Leu-Lys-*NH₂ (XIIIa) (SEQ ID NO. 13),
wherein the amino acids in D configuration are indicated in italics, amino acids in L-configuration are written in normal font type, H- indicates a free N-terminal amino group, -NH₂ indicates an amidated C-terminal carboxyl group of the peptides (-C(O)NH₂), and Met(O) is methionine sulfoxide.

The peptides of the formulae VII, X, XI and XII are in the configuration of all-L-isomer, i.e., all amino acids are in L-configuration. The peptides of the formulae VIIa, Xa, XIa and XIIa have an identical sequence of amino acids as VII, X, XI and XII, respectively, but the amino acids are in the configuration of all-D-isomer, i.e. peptides VIIa, Xa, XIa and XIIa are composed only of D-amino acids. The peptides of formula XIII and of formula XIIIa have an identical sequence of amino acids, but are in mutually reverse configurations, i.e., in XIII, Leu in position 7 is in D-configuration and the remaining amino acid residues are in L-configuration, and in formula XIIIa, Leu in position 7 is in L-configuration and the remaining amino acid residues are in D-configuration.

The invention further provides peptides of specific formulas I to XIII and their above described enantiomers for use in the treatment of musculoskeletal infections in orthopaedics and traumatology, particularly for the treatment of infectious diseases of bones and joints or infected surrounding tissue, and/or for prevention or eliminating the infectious agent from the joint and bone replacement, and sealants, and generally to prevent infectious complications occurring after implantation of joint replacements and after osteosynthesis.

Preferably, peptides of formulas I to XIII and their above described enantiomers in a mixture with the carrier material are used for treating an infectious disease of bones, or the infected surrounding soft tissue and/or for prevention or elimination of the infectious agent from the joint and bone replacement, and sealants, or generally to prevent infectious complications occurring after implantation of joint replacements and after osteosynthesis.

The carrier material can be preferably selected from calcium phosphate, calcium sulfate, bioactive glass, apatite-wollastonite ceramic bioglass, hydrogel, collagen, bone graft, bone cement, or synthetic polymers such as polymethyl methacrylate, copolymer of methyl methacrylate and hydroxyethyl methacrylate, polyanhydride, polylactide, polyglycolide, a copolymer of hydroxybutyrate and hydroxyvalerate, polyhydroxyalkanoate, polycaprolactone or gelatine sponge with glycerine, or composites consisting of polymers and mineral carriers.

Subject matter of the invention also involves peptides of the formulae I to XIII and their above described enantiomers, for production of drugs for treatment or prevention of infectious diseases of bones or infected surrounding tissues, and/or for prevention or elimination of the infectious agent from joint and bone replacements and sealants, and for the prevention of infection of orthopaedic implants.

Subject matter of the invention also involves a pharmaceutical composition which contains therapeutically effective amount of at least one peptide of the general formula I to XIII or being their above described enantiomer, optionally a second active ingredient and at least one pharmaceutically acceptable carrier, filler and/or diluent.

An aspect of the invention is that the above-mentioned composition contains an antibiotic or antimycotic agent as the second active ingredient.

Another aspect of the invention is that the above-mentioned pharmaceutical composition is intended for treatment or prevention of infectious diseases of bones or infected surrounding tissue, and/or prevention or elimination of the infectious agent from joint and bone replacements and sealants, and for the prevention of the infection of orthopaedic implants.

Synthetic peptides of formulae I to XIII and their above described enantiomers can be included among halictins, named after the natural peptide halictin II [22]. Appropriate substitution of some amino acids within its sequence, and surprisingly also shortening by one amino acid at the N-terminus, a substantial increase in antimicrobial activity of the new peptides, and a reduction in their haemolytic activity was unexpectedly achieved. They have therefore become usable for applications in the presence of eukaryotic cells, which now allows their use as active ingredients in human or veterinary drugs. Analogues thus obtained are shown in Table 1; values of antimicrobial and haemolytic activities resulting from the performed substitutions are then given in Table 2. This group of peptides derived from halictin II surprisingly showed a significant antimicrobial effect in infected bone tissue. It was also demonstrated that other seemingly very little substitutions to the sequence of these peptides lead to analogues that do not show a significant effect in the infected bone tissue, and in addition, their use is ruled out by undesirable haemolytic activity.
Peptides from the invention I-XIII and their above described enantiomers may advantageously be combined with a local carrier. Bacterial load of the infected area of the bone was significantly lower after treatment with the peptides with a carrier than in an infected area where only the local carrier without peptides was applied or only the local carrier with antibiotics such as vancomycin or gentamicin. If bone cement based on polymethyl methacrylate was used as the local carrier in combination with any of the peptides I-XIII or of their above described enantiomers and the thus produced material was implanted into the previously infected bone marrow, formation of microbial biofilm on its surface was prevented. In contrast, biofilm was always formed in a cement implant, which did not contain any peptide. In some instances a biofilm was formed even on implants prepared from Vancogenx® cement which contained antibiotics vancomycin and gentamycin from the manufacturer. In another embodiment, cements were first implanted into uninfected bone marrow before bone was infected with microbes through a slit made aside from these implants. Microbes from the slit penetrated the whole bone marrow and colonized the implant, which did not contain any peptide, with consequent formation of a biofilm on its surface. Colonization of the implant and biofilm formation was effectively prevented by using an implant containing peptide I to XIII or their above described enantiomer of the invention.

Practical use of new antimicrobial peptides will involve primarily the local treatment of infections of both bacterial and yeast origin. Their application will be beneficial for example in orthopaedics for the treatment of osteomyelitis (bone infection), to prevent infection of orthopaedic implants, on which microbial biofilms may be formed, but also in traumatology in treatment and prevention of infected bone and surrounding soft tissues. Newly synthesized antimicrobial peptides of formulae I-XIII and their above described enantiomers demonstrated high efficiency in tests involving the above mentioned indications.
Topical application of antimicrobial peptides include their incorporation into local carriers, including bone cements and implants used in orthopaedics.

This approach provides a promising therapeutic method for example for the treatment of osteomyelitis and a method for preventing the formation of microbial biofilms on the surface of the implants used in orthopaedics. The advantage of using antimicrobial peptides incorporated into a carrier is mainly in achieving high local concentrations of peptides without systemic toxicity, since peptides are then easily metabolized in the body. A suitable carrier is selected from several groups including natural materials based on collagen, fibrin or thrombin, blood coagulate, or demineralized bone matrix or bone grafts. Furthermore, mineral carriers such as calcium phosphate, calcium sulphate, hydroxyapatite, nano-hydroxyapatite and beta-tricalcium phosphate, as well as bioactive glass and apatite-wollastonite ceramic bioglass can be used. Synthetic polymers, such as polymethylmethacrylate (PMMA), PMMA/polyhydroxyethylmethacrylate (PHEMA), polyanhydride, polylactide, polyglycolide, polyhydroxybutyrate-co-hydroxyvalerate, polyhydroxyalkanoate, polycaprolactone and gelatin sponge with glycerine, or composite of polymers and mineral carriers are also often used. Suitable carrier is selected according to the method of application and indication of the type of the musculoskeletal infection. It is best to use carriers available from commercial sources for immediate use.
In addition to treatment of infectious diseases of bones (osteomyelitis) and the accompanying complications, another utilization of new antimicrobial peptides of the invention involves treatment or prevention of the surrounding infected tissues of musculoskeletal system, but also the prevention (treatment) of infectious complications when using joint replacement and osteosynthesis, associated with the formation of microbial biofilms on their surfaces.

The term "pharmaceutically acceptable"refers to those compounds, which are suitable for use in contact with the tissues of patients without undue toxicity, irritation and allergic response, are commensurate with a reasonable benefit/risk ratio, and are effective for their intended use.

To verify the antimicrobial effect of the present peptides on the infected bone, a model of induced osteomyelitis was developed in laboratory conditions. It uses femur heads, taken from patients during hip replacement surgery. These heads were provided as discarded from the bone tissue bank in Motol University Hospital in Prague. Effect of the peptides against infection in induced osteomyelitis model is further described in Example 4 and the results obtained are summarized in Table 3.

### Description of the Figures

Fig. 1 shows a joint head with infected holes filled with a carrier (ChronOS Inject) containing a peptide, an antibiotic and the carrier itself. Unfilled infected hole was kept as a control in this case.
Fig. 2 shows a joint head with infected holes filled with polymethyl methacrylate bone cement (Palacos®r) without peptide (1, 2); containing the peptide (3, 4); and containing vancomycin (5).
Figure 3 shows an effect of peptide VIII on the eradication of the infection inside the spongy bone tissue: Panel A, no peptide, panel B, peptide VIII. In lanes 1 to 4 are shown bacterial colonies in tenfold serial dilutions. For the quantification see Table 5.

### Examples

### List of abbreviations:

- Fmoc: 9-fluorenylmethyloxycarbonyl
- MBHA resin: 4-methylbenzhydrylamine resin
- TFA: trifluoroacetic acid
- TIS: triisopropylsilane
- HPLC: high performance liquid chromatography
- BHI medium: brain-heart infusion medium
- YPG medium: yeast-peptone-glucose medium
- LB medium: Luria Bertani medium
- CFU: colony forming units

### Example 1

### Synthesis of the compounds of the formulae I to XIII and their specific enantiomers

Antimicrobial peptides of formulas I to XIII and their specific enantiomers were synthesized by the method of solid phase peptide synthesis (SPPS). The synthesis was performed manually in 5 ml polypropylene syringes with a polypropylene filter in the bottom of the syringe, using Fmoc-chemistry protocol [23]. Rink Amide MBHA resin (IRIS Biotech GmbH, Germany) with substitution of 0.7 mmol.g⁻¹ was used as the solid carrier. Amino acids whose amine function was protected by Fmoc, were condensed in a 4-fold excess in DMF and N,N'-diisopropylcarbodiimid in the presence of 1-hydroxybezotriazole was used for the condensation. After each condensation step, the Fmoc-protecting group was cleaved with 20% (vol./vol.) piperidine in DMF.

Crude peptides I-XIII and their specific enantiomers were obtained by cleavage from the resin and simultaneous deprotection of the protecting groups with TFA/1,2-ethanedithiol/H₂O/thioanisole/TIS (90 : 2.5 : 2.5 : 3 : 2) for 3.5 hours and subsequent precipitation with *tert-*butyl methyl ether. When the peptide did not contain methionine, a simpler mixture of TFA/H₂O/TIS (95 : 2.5 : 2.5) was used for the cleavage (volume fractions are given). This procedure yielded 90 to 100 mg of crude peptides. A portion of this material (30 mg) in each case was purified by preparative HPLC on a Vydac C-18 column (250 x 10 mm) at a flow rate of 3.0 ml/min, using a gradient from 5% acetonitrile/water/0.1% TFA up to 70% acetonitrile/water/0.1% TFA (percentages by volume are given).

This yielded 10-15 mg of HPLC pure peptide whose identity was verified by mass spectrometry, as shown in Table 1.

**Table 1. Amino acid sequences and molecular masses of the peptides I - XIII**

| | Peptide sequence ^{a)} | Molecular mass (Da) | |
|---|---|---|---|
| | | Calculated | Found |
| I | H-Lys-Trp-Met-Lys-Leu-Leu-Lys-Lys-Ile-Leu-Lys-NH₂ | 1426,96 | 1427,0 |
| II | H*-Lys-Trp-Met-Lys-Leu-Leu-Lys-Lys-Ile-Leu-Lys-*NH₂ | 1426,96 | 1427,0 |
| III | H-Gly-Lys-Trp-Met-Lys-Leu-Leu-Lys-Lys-Ile-Leu-Lys-NH₂ | 1483,98 | 1484,0 |
| IV | H-Gly-Lys-*Trp-Met-Lys-Leu-Leu-Lys-Lys-Ile-Leu-Lys-*NH₂ | 1483,98 | 1484,3 |
| V | H-βAla-Lys-Trp-Met-Lys-Leu-Leu-Lys-Lys-Ile-Leu-Lys-NH₂ | 1497,99 | 1498,5 |
| VI | H-βAla-*Lys-Trp-Met-Lys-Leu-Leu-Lys-Lys-Ile-Leu-Lys*-NH₂ | 1497,99 | 1498,2 |
| VII | H-Gly-Lys-Trp-Leu-Lys-Leu-Leu-Lys-Lys-Ile-Leu-Lys-NH₂ | 1466,02 | 1466,7 |
| VIII | H-Gly-Lys-Trp-Val-Lys-Leu-Leu-Lys-Lys-Ile-Leu-Lys-NH₂ | 1452,01 | 1452,0 |
| IX | H-Gly-*Lys-Trp-Val-Lys-Leu-Leu-Lys-Lys-Ile-Leu-Lys*-NH₂ | 1452,01 | 1452,0 |
| X | H-Gly-Lys-Trp-Lys-Lys-Leu-Leu-Lys-Lys-Ile-Leu-Lys-NH₂ | 1481,03 | 1481,0 |
| XI | H-Gly-Lys-Trp-Met-Lys-Met-Leu-Lys-Lys-Ile-Leu-Lys-NH₂ | 1501,94 | 1501,9 |
| XII | | 1499.97 | 1480,0 |
| XIII | H-Gly-Lys-Trp-Met-Lys-Leu-*Leu*-Lys-Lys-Ile-Leu-Lys-NH₂ | 1483,98 | 1484,0 |

| | | | |
|---|---|---|---|
| ^{a}) amino acids in the D configuration are indicated in italics; amino acids in the L-configuration are in normal type, Met(O) is methionine sulfoxide | | | |

### Example 2

### Determination of an antimicrobial activity

Gram-positive bacteria (*Bacillus subtilis, Staphylococcus aureus, Staphylococcus epidermidis* and *Enterococcus faecalis*)*,* Gram-negative bacteria (*Escherichia coli* and *Pseudomonas aeruginosa*) and the yeast (*Candida albicans*) were grown to exponential phase and then were diluted with fresh medium. BHI medium (Oxoid, Great Britain) was used for *Enterococcus faecalis*; YPD medium containing yeast extract, peptone and glucose was used for *Candida albicans* and LB medium (Sigma, Czech Republic) was used for the remaining microorganisms. Then the microorganisms were added to the solutions of the tested antimicrobial peptides in LB medium in multi-well plates, so that the final peptide concentration it the wells was in the range of 0.5 to 100 µmοl.1⁻¹. The plates were incubated at 37° C for 20 h while being continuously shaken in a Bioscreen C (Oy Growth Curves AB Ltd., Helsinki, Finland). Absorbance was measured at 540 nm every 15 min. Antimicrobial activity (Table 2) was determined as a quantitative minimum inhibitory concentration (MIC) by observing bacterial growth in the presence of different concentrations of tested peptides. In the experiments, ca 10⁵ CFU of bacteria or candida per well were used. Tetracycline in a concentration range of 0.5 - 100 µmol.1⁻¹ was used as a standard.

Clinical isolates of bacteria were obtained from University Hospital in Motol, Prague, Czech Republic and from Liberec Regional Hospital, Czech Republic. Methicillin resistant *Staphylococcus aureus* (MRSA) 6271 and *Pseudomonas aeruginosa* 5482 were from the Czech National Collection of Type Cultures, the National Institute of Public Health, Prague, Czech Republic. Other bacteria were from the Czech Collection of Microorganisms, Brno, Czech Republic. *Candida albicans* came from the Faculty of Medicine, Palacky University, Olomouc, Czech Republic. *Candida tropicalis* ATCC 750 and *Candida glabrata* DSY 565 were obtained from the Institute of Physiology, Academy of Sciences of the Czech Republic.

### Example 3

### Determination of a haemolytic activity

The peptides in the concentrations 2-400 µmol.1⁻¹ were incubated with the suspension of human red blood cells (5% v/v) for 1 h at 37 °C in a physiological solution at a final volume of 0.2 ml. The samples were then centrifuged for 5 min at 250 g. The absorbance of the supernatant was determined at 540 nm. 0.2% (v/v) TRITON X100 was used as a control for 100% haemolysis. The results of tests are shown in Table 2. Haemolytic activity is expressed as the peptide concentration required for lysis of 50% of human erythrocytes in the assay (LC₅₀).

**Table 2. Antimicrobial and haemolytic activity of I up to XIII peptides and tetracycline.**

| Peptide | Antimicrobial activity MIC (µmol.l⁻¹) | | | | | | | | | | Hemolytic activity LC₅₀ (µmol.l⁻¹) |
|---|---|---|---|---|---|---|---|---|---|---|---|
| | *B.s.* | *MRSA* (clin. isolate Liberec) | *MRSA 6271* | *MRSA* (clin. isolate Motol) | *S.e.* (clin. isolate Motol) | *E.f.* (clin. isolate Motol) | *E.c.* | *P.a.* (clin. isolate Liberec) | *P.a. 5482* | *C.a.* | |
| I | 2,3 | 8,0 | 13,5 | 17,7 | 3,2 | >100 | 12,7 | 5,8 | 2,9 | 9,5 | >400 |
| II | 1,8 | 14,0 | 19,3 | 14,0 | 3,5 | 36 | 3,5 | 18,0 | 4,5 | 11,3 | >400 |
| III | 1,8 | 3,6 | 6,3 | 5,7 | 2,3 | 13,3 | 9,0 | 10,0 | 2,8 | 5,7 | 339 |
| IV | 1,4 | 5,3 | 11,3 | 9,7 | 2,0 | 12,7 | 3,0 | 12,5 | 4,5 | 5,7 | >400 |
| V | 1,8 | 5,7 | 11,3 | 10,0 | 2,8 | 59,0 | 6,8 | 7,0 | 2,8 | 11,3 | 270 |
| VI | 1,6 | 8,0 | 16,0 | 16,0 | 2,5 | 32,0 | 3,2 | 8,0 | 3,2 | 5,3 | >400 |
| VII | 2,0 | 8,0 | 10,0 | 10,0 | 2,2 | 50,7 | 5,0 | 10,7 | 4,0 | 16,0 | 196 |
| VIII | 2,0 | 5,0 | 9,0 | 10,0 | 2,5 | >100 | 6,3 | 12,7 | 3,0 | 6,3 | >400 |
| IX | n.t | 15,0 | 22,0 | 22,0 | 4,2 | 25,3 | n.t. | 25,3 | 4,5 | 12,5 | >400 |
| X | 2,4 | 11,5 | 29,7 | 25,3 | 4,7 | >100 | 11,5 | 9,5 | 2,9 | 9,5 | >400 |
| XI | 2,5 | 10,0 | 20,0 | 20,0 | 3,2 | >100 | 20,0 | 12,7 | 5,3 | 12,7 | >>200 |
| XII | 2,5 | 11,5 | 64,0 | 25,3 | 5,0 | >100 | 26,7 | 22,1 | 5,0 | 21,3 | >400 |
| XIII | 2,8 | 13,3 | 18,7 | 32,0 | 4,0 | >100 | 10,0 | 32,0 | 5,0 | 8,0 | >400 |
| Tetracyklin | 16,0 | 1,1 | 0,9 | 2,0 | >100 | 40,0 | 2,7 | 70,4 | 28,7 | - | - |

| | | | | | | | | | | | |
|---|---|---|---|---|---|---|---|---|---|---|---|
| *B.s., Bacillus subtilis; MRSA,* methicilin-resistent *Staphylococcus aureus; S.e., Staphylococcus epidermidis; E.f., Enterococcus faecalis; E.c., Escherichia coli; P.a., Pseudomonas aeruginosa; C.a., Candida albicans;* n.t. = not tested | | | | | | | | | | | |

### Example 4

### Effect of selected peptides on a focus of infection in the bone tissue

3-4 holes of a diameter of 5 mm and a depth of 10 mm are drilled with a surgical spoon into a femur head under sterile conditions (spongy part of the bone in the resection site). Thereafter, this bone graft is put into a beaker of appropriate size and lined with sterile gauze squares. The gauze is moistened with sterile physiological saline to prevent drying of the bone. Then, suspension of multiplied microbes in an appropriate medium (50-100 µl) at a concentration of 10⁷ to 10⁹ CFU is sequentially applied to the prepared holes in the bone graft and allowed to incubate for 1-2 days. This results in an induced osteomyelitis model. Beaker with the infected bone graft is covered with a sterile Petri dish. During the experiment, induced osteomyelitis models are kept protected from light at room temperature.

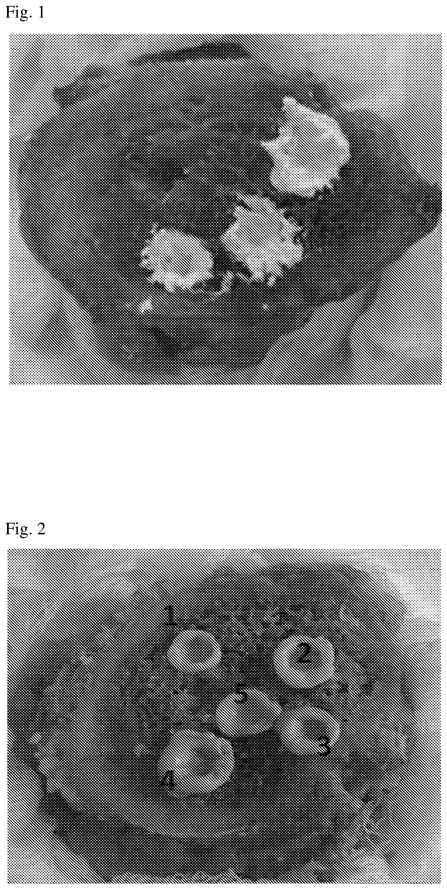

After the incubation, the infected holes in the bone are filled with a mixture of the local carrier, and a peptide selected from the series of peptides of formulae I to XIII or their specific enantiomers (Figure 1). For comparison, one of the holes is filled with the local carrier without peptide, or the carrier filled with antibiotic (vancomycin or gentamicin). A commercially available two-component material supplied by Synthes (Synthes GmbH, Switzerland) under the name ChronOS Inject was used as the carrier. Its solid component comprises a mixture of calcium phosphates CaHPO₄.2H₂O and Ca₃(PO₄)₂ and additives, the liquid component is 0.5% (vol./vol.) solution of hyaluronic acid. For preparation of a paste for filling the hole, 200 to 400 mg of the solid carrier component is used, 5 mg - 20 mg of peptide is stirred in, preferably 10 mg (or a mixture of L- and D-form of the peptide in the ratio 4:1), or 7 mg or vancomycin, or 5.5 mg gentamicin, or 5.4 mg ceftriaxone; and then the liquid component is stirred into this mixture in an amount of 60 - 80 µl to 200 mg of the solid component, and all of that is thoroughly mixed (a paste without peptide for the control experiment is prepared by the same procedure) .

After two days, the filling is thoroughly removed with a surgical spoon and empty holes are wiped with moistened sterile cotton swab, which is then extracted into 400 µl physiological saline. The extract is diluted in a tenfold serial dilution and each diluted suspension is cultured on agar in Petri dishes; the culture is evaluated on the following day. The result of the experiment is evaluated by comparing the number of bacterial colonies from the swab from infected holes where the peptide was released from the carrier, with the number of colonies from the swab from the infected hole that was filled with carrier only without any peptide (Table 3). The result of the peptide effect is also compared with the result of the effect of antibiotics. In some cases, microbiological examination of the bone tissue taken with a surgical spoon from a deeper area of the drilled holes is performed.
Used femur heads are autoclaved to deactivate the infection and disposed of as clinical waste on the pathology of the Motol University Hospital.

**Table 3. Evaluation of the effect of antimicrobial peptides released from the carrier on an infection in the bone graft. Effect of antimicrobial activity is expressed in the number of CFU (colony-forming units) in the infected hole after swabbing.**

| Bacteria | **CFU/hole** | | |
|---|---|---|---|
| | Carrier without peptide | Carrier with peptide No. ^{a)} | Carrier with an antibiotic |
| *MRSA* (clin. isolate Motol) | ChronOS (200 mg) | III (8 mg)+IV (2 mg) | Vancomycin (7 mg) |
| | **5,6x10⁶** | **2,4x10⁵** | **2,0x10⁶** |
| *MRSA* (clin. isolate Motol) | ChronOS (200 mg) | III (8 mg)+IV (2 mg) | Gentamycin (5.2 mg) |
| | **7,0x10⁷** | **5,4x10²** | **9,2x10⁵** |
| *MRSA* (clin. isolate Motol) | ChronOS (200 mg) | V (10 mg) | N/A |
| | **4,8x10⁶** | **4,0** | |
| *MRSA* (clin. isolate Liberec) | ChronOS (200 mg) | I (10 mg)/III (10 mg)⁾ | N/A |
| | **3,4x10⁷** | **2,3x10⁵ / 2,3x10⁵** | |
| *MRSA* (clin. isolate Liberec) | ChronOS (300 mg) | III (8.4 mg)+IV (2.5 mg) | N/A |
| | **2,1x10⁵** | **0** | |
| *MRSA* (clin. isolate Liberec) | ChronOS (300 mg) | III (8.0 mg)+IV (2.0 mg) | N/A |
| | **1,2x10⁶** | **0** | |
| *MRSA* (clin. isolate Liberec) | ChronOS (300 mg) | III (4.5 mg)+IV (1.2 mg) | N/A |
| | **1,6x10⁶** | **4,0x10²** | |
| *MRSA* (clin. isolate Liberec) | ChroOS (400 mg) | III (10.7 mg)+IV (2.7 mg) | N/A |
| | **2,8x10⁶** | **0** | |
| *MRSA* (clin. isolate Liberec) | ChronOS (200 mg) | III (8 mg)+IV (2 mg) | Vancomycin (7 mg) |
| | **1,2x10⁷** | **4,8x10³** | **6,0x10⁶** |
| *S. aureus* (clin. isolate Liberec) | ChronOS (200 mg) | II (10 mg) | Vancomycin (10 mg) |
| | **1,4x10⁷** | **1,8 x10⁴** | **1,4x10⁷** |
| *S. aureus* (clin. isolate Liberec) | ChronOS (200 mg) | XI (10 mg) | Ceftriaxon (5 mg) |
| | **2,0x10⁶** | **2,5 x10³** | **4,1x10⁶** |
| *S. aureus* (klinický izolát Liberec) | ChronOS (200 mg) | I (10 mg)/VIII (10 mg) | Gentamicin (10 mg) |
| | **3,9x10⁶** | **1,3x10⁴** / **0** | **2,9x10⁵** |
| *MRSA 6271* | ChronOS (200 mg) | III (10 mg)/III (5 mg) | N/A |
| | **7,0x10⁷** | **7,6x10²** / **1,9x10²** | |
| *MRSA 6271* | ChronOS (200 mg) | III (8 mg)+IV(2 mg) | Vancomycin (7 mg) |
| | **4x10⁵** | **2x10³** | **2x10⁵** |
| *S. epidermidis* (clin. isolate Motol) | ChronOS (200 mg) | VII (10 mg) | Gentamicin (5.2 mg) |
| | **7,2x10⁴** | **1,2 x10²** | **6,0x10³** |
| *S. epidermidis* (clin. isolate Motol) | ChronOS (300 mg) | III (10 mg) / VIII (10 mg) / IX (10 mg) | N/A |
| | **1,2x10⁵** | **0** in all cases | |
| *P. aeruginosa* 5482 | ChronOS (200 mg) | III (8 mg)+IV (2 mg) | N/A |
| | | **4,0x10⁴** | |
| | **1,0x10⁹** | V (9 mg) | |
| | | **2,0x10⁵** | |

| | | | |
|---|---|---|---|
| ^{a)} + = a mixture of two peptides was applied into the selected hole, / = various holes in the bone sample were treated with various peptides or a peptide in varying quantities | | | |

It is apparent from this table that selected test peptides from peptides I to XIII, both individually and in combination of two peptides, after release from the carrier to the bone graft, demonstrated significant antimicrobial effect in induced osteomyelitis tests (Figure 1). In Table 3, this effect is expressed by the number of bacterial colonies (CFU) that correspond to the residual infection in the bone graft after exposure to the peptide. For all peptides and their combinations tested, the number of CFU was two to six orders of magnitude lower than in the case of application of the carrier alone without peptide. The effect of peptide was even two to three orders of magnitude higher than the effect of simultaneously tested antibiotics, particularly vancomycin or gentamicin.

Similar results were achieved using a carrier based on BonAlive bioactive glass (BonAlive Biomaterials Ltd, Finland).

To verify the effect of our peptides for the prevention of biofilm formation on the implant surface, a model of induced biofilm formation in bone tissue in laboratory conditions was developed, using femur heads similar to the above. Effect of the peptides against biofilm formation on the surface of the implant made of bone cement based on polymethyl methacrylate in a model of induced osteomyelitis is further described in Example 5 and the results obtained are summarized in Table 4.

### Example 5

### Effect of selected peptides against biofilm formation on the implant surface

Model of induced osteomyelitis using femur heads is prepared in the same manner as in Example 4. Bone cement (Palacos®r, Hereaus Medical GmbH, Germany) containing the peptide is prepared by mixing the peptide from the series of peptides I to XIII or their above described enantiomers with a powdered polymeric component of cement (poly-methylacrylate, poly-methyl methacrylate, benzoyl peroxide) and subsequently with a liquid monomer component (methyl methacrylate, N, N-dimethyl-p-toluidine). For the preparation of a paste for filling the infected holes, 125 mg powder component of cement is used, 5 mg - 20 mg (preferably 10 mg) of peptide is stirred in, or 7 mg of vancomycin, or 10 mg of fluconazole, or 10 mg of amphotericin B; then the liquid component is stirred into this mixture in an amount of 65 - 70 µl to 125 mg of the solid component and all of that is thoroughly mixed (a paste without peptide for the comparative experiment is prepared by the same procedure). The resulting elastic paste is placed into a 2 ml plastic syringe (without needle) and pressed into the infected holes in the spongy part of the bone, where it polymerizes and hardens. For comparison, other similarly infected holes are filled with cement containing only an antibiotic or antimycotic agent. For comparison, a Vancogenx® cement (Tecresa S. p. A., Italy) is used, made from a mixture of a powdered polymer component containing vancomycin and gentamicin. The mixture in an amount of 125 mg is mixed with liquid monomer (50-60 µl) and then pressed into the openings as described above. In another embodiment, the cements are applied to uninfected holes, allowed to harden, and then microbes are pipetted into the hole excavated between implants. From there they penetrate through bone tissue and settle on the implants with consequent formation of biofilms.

After two days, implants are removed with metal spikes and tweezers, mechanically cleaned from debris of bone tissue, and washed four times with physiological saline (0.5 ml) in plastic tubes to remove any residual planktonic microbes or bone cells from their surface. The implants in closed tubes with 0.4 ml saline are then sonicated four times for 3 min with occasional shaking. The of microbial biofilm on the surface of the implant is thus disrupted and microbes are loosed into the physiological saline. The resulting suspension is subsequently diluted using the tenfold serial dilution for culturing on agar.

The result of the experiment is evaluated by comparing the number of microbial colonies corresponding to the number of microbes released from the implant surface, in which the peptide was incorporated, to the number of colonies from the implant surface, which did not contain the peptide (Table 4). The result of the peptide effect to prevent biofilm formation compared to the result of the effect of antibiotics or antifungal agents is also evaluated. Used femur heads are autoclaved to deactivate the infection and disposed as described in example 4.

**Table 4. Evaluation of the effect of antimicrobial peptides on the formation of microbial biofilm on the surface of the implant made of the bone cement. The effect of the peptides is expressed as the number of microbial colonies (CFU) corresponding to the number of microbes released from the biofilm attached to the surface of the implant.**

| Microbe | **CFU/implant** | | |
|---|---|---|---|
| | Cement without peptide | Cement with peptide No. | Cement + an antibiotic or antimycotic agent |
| *MRSA* (clin. isolate Motol) | | **III** | Palacos®r + Vancomycin |
| | **3,4x10⁵** | **0** | **4,0 x 10⁶** |
| *MRSA* (clin. isolate Motol) | | **I** | Vancogenx® |
| | **2,6x10⁶** | **0** | **0** |
| MRSA (clin. isolate Liberec) | **8,8x10³** | **I** | N/A |
| | | **0** | |
| MRSA (clin. isolate Liberec) | **7,0x10⁶** | **XI** | N/A |
| | | **0** | |
| *MRSA 6271* | | **III** | Vancogenx® |
| | N/A | **5.1 x 10³** | 8.0 **x 10⁶** |
| *MRSA 6271* | | **III** | Vancogenx® |
| | N/A | **0** | **8.4x10⁵** |
| *MRSA 6271* | | **I** | Vancogenx® |
| | **1.1x10⁶** | **0** | **3.0x10⁵** |
| *MRSA 6271* | | **XII** | N/A |
| | **6.0x10⁴** | **0** | |
| *S. epidermidis* (clin. isolate Motol) | | **I** | Vancogenx® |
| | **4.0x10⁶** | **1,1x10³** | **4,0x10⁶** |
| *Enterococcus faecalis* (clin. isolate Motol) | | **III** | Palacos®r + Vancomycin |
| | **2.8x10⁶** | **0** | **4,0x10⁶** |
| *Enterococcus faecalis* (clin. isolate Motol) | | **III** | N/A |
| | **2.9x10⁵** | **0** | |
| *P. aeruginosa* 5482 | | **III** | N/A |
| | **2.8x10⁶** | **2.6x10¹** | |
| *P. aeruginosa* (clin. isolate Liberec) | | **III** | Vancogenx® |
| | N/A | **1.1x10³** | **4 x 10⁶** |
| *P. aeruginosa* 5482 | | **I** | Vancogenx® |
| | **6x10⁵** | **7.0x10²** | **0** |
| *Candida tropicalis* ATCC 750 | | **III** | Palacos®r + Amfotericin |
| | **3.7x10⁵** | **0** | **1.3 x10⁶** |
| *Candida albicans* (clin. isolate Olomouc) | | **XIII** | Palacos®r + Amfotericin |
| | **8.0x10⁴** | **0** | **0** |
| *Candida albicans* (clin. isolate Olomouc) | | **III / I** | N/A |
| | **6.0x10³** | **2 / 6.8x10¹** | |
| *Candida albicans* ATCC MYA-2876 | | **XIII** | Palacos®r + Fluconazol |
| | **4.4x10⁵** | **6.0x10³** | **3.6x10⁴** |
| *Candida glabrata* DSY565 | **1.3x10⁶** | **XIII** | Palacos®r + Clotrimazol |
| | | **2.4x10³** | **1.2x10⁶** |

It is apparent from this table that selected test peptides from the group of peptides I to XIII incorporated into bone cements show a significant effect of preventing formation of a microbial biofilm on its surface in assays of induced osteomyelitis (Figure 2). In Table 4, this effect is expressed in the number of bacterial colonies (CFU) that correspond to the residual infection deposited on the surface of cement with incorporated peptide. The number of CFUs at all tested peptides or their combinations was always five to six orders of magnitude lower than in the case of using cement alone without peptide. The effect of the peptides was in some cases by several orders of magnitude higher than the effect of simultaneously tested cements containing vancomycin and one order of magnitude higher than the effect of simultaneously tested cement Vancogenx®, containing mixture of vancomycin and gentamicin from the manufacturer.

### Example 6

### Comparison of the effect of two similar halictin peptides for biofilm formation on the implant surface

The experiment was performed under similar conditions as described in Example 5. Here, 100 mg of the powder cement component was used to prepare a paste, which was mixed with in 3.5 mg of peptide XIII and subsequently 52 µl of liquid component. The resulting paste was thoroughly mixed and then pressed into a hole prepared in the spongy part of the bone. To a next hole in the spongy part of the same bone a paste was pressed, containing comparative peptide Hal 2/4 (3.5 mg) with the sequence H-Lys-Trp-Met-Ser-Leu-Leu-Lys-Lys-Ile-Leu-Lys-NH₂, which was also prepared from 100 mg of the powder component and 52 µl of the liquid component. The third hole was filled with paste without peptide prepared from the same amount of components. The *Candida albicans* yeast (clinical isolate Olomouc), grown in YPG, was pipetted in the hole excavated between implants equidistant (approximately 1 cm) from each. From there, the yeast penetrated through the bone tissue and was deposited on the implant with consequent formation of biofilms.

After two days, the implants were removed, mechanically cleaned of bone tissue residues and washed four times with physiological saline (0.5 ml) in plastic tubes to remove planktonic microbes or residues of bone cells from their surfaces. Then the implants in closed tubes with 0.4 ml of saline were treated ultrasonically four times for three minutes with occasional shaking. This way, microbial biofilm on the surface of the implant was disrupted and microbes loosed into the physiological saline. The resulting suspension was then diluted in a decimal dilution series for culturing on agar.
The result of the experiment was evaluated by comparing the number of microbial colonies (CFU), corresponding to the number of microbes released from the surfaces of implants.
The number of colonies from the implant surface, which contained no peptide was 1.8 **×** 10³ CFU/implant and the number of colonies from the surface of the implant, containing a Hal 4/2 peptide of the above sequence, was 2.8 **×** 10² CFU/implant. In contrast, the number of colonies on an implant containing peptide XIII was zero.

### Example 7

### Effect of peptide VIII on the eradication of the infection inside the spongy bone tissue

Experiments was carried out similarly as described above using two graft bones. The suspension of *Staphylococcus aureus* (MRSA 6271) in LB medium (50 - 100 µl) in the concentration 10⁹ CFU/ml was pipetted into two holes drilled in each bone graft. On the next day the one hole in one bone graft was filled with the carrier (200 mg) blended with peptide VIII (10 mg). The second hole was filled with carrier alone (200 mg). In the second bone graft the two holes were filled identically.

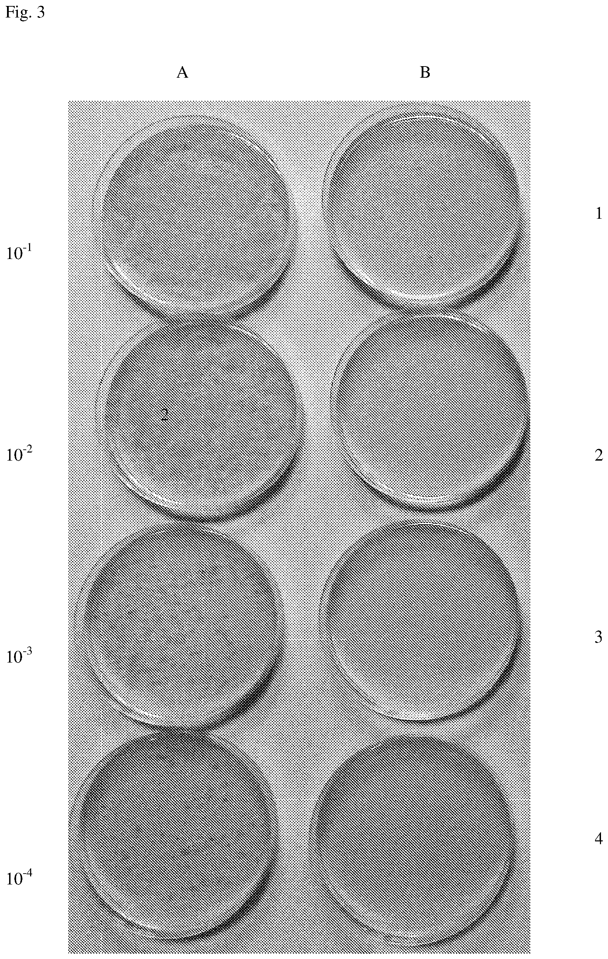

The experiments were evaluated after two days of the treatment by the comparison of the number of bacterial colonies (CFU/ml) related to the infected hole in which the peptide was released from the carrier and acted against the infection, against the number of the colonies related to the infected hole which was filled only with the carrier alone. The results of these two independent experiments which are summarized in Table 5 clearly show high efficacy of the peptide VIII. Its efficacy is also shown in the Figure 3.

**Table 5. Effect of peptide VIII on the eradication of the infection inside the spongy bone tissue Carried out in two independent experiments.**

| Bacterium | CFU/ hole | |
|---|---|---|
| | No peptide | Peptide VIII |
| MRSA 6271 | 1.2x10⁷ | 0 |
| MRSA 6271 | 3.6x10⁶ | 4.8x10² |

### Industrial applicability

Practical use of new antimicrobial peptides derived from halictin II will involve local treatment of infections of both bacterial and yeast origin. Their application will be beneficial for example in orthopaedics for the treatment of osteomyelitis (bone infection), to prevent infection of orthopaedic implants, and microbial biofilm formation on them, but also in traumatology in treatment and prevention of infections of bones and surrounding soft tissues. Newly synthesized antimicrobial peptides of formulae I-XIII and their specific enantiomers demonstrated high efficiency in tests involving the aforementioned indications, which was several orders of magnitude higher in the model of induced osteomyelitis than the efficiency of antibiotics such as vancomycin or gentamicin, and they also prevented the formation of microbial biofilms on surfaces of model implants made of bone cement.

### References

1. Melicherčík P., Jahoda D., Landor I., Pokorný D., Judl T., Sosna A. Lokální nosiče antibiotik. Ortopedie 4, 187-193 (2011)
2. Melicherčík P., Jahoda D., Barták V., Klapková E., Nyč O., Pokorný D. In vitro assessment of vancomycin released from bone grafts. Acta Chir. Orthop. Traum. Čech. 77, 411-415 (2010)
3. Melicherčík P., Jahoda D., Nyč O., Klapková E., Barták V., Landor I., Pokorný D., Judl T., Sosna A. Bone grafts as a vancomycin carrier for local therapy of resistant infections. Folia Microbiol. 57, 459-462 (2012)
4. Aiken S.S., Cooper J.J., Florance H., Robinson M.T., Michell S. Local release of antibiotics for surgical site infection. Management using high-purity calcium sulfate: an in vitro elution study. Surg. Infect. 16, 54-61 (2015)
5. Romano C.L., Toscano M., Romano D., Drago L. Antibiofilm agents and implant-related infections in orthopeadics: where are we? J. Chemother. 25, 67-80 (2013)
6. van de Belt H., Neut D., Schenk W., van Horn J.R., van der Mei H.C., Busscher H.J. Staphylococcus aureus biofilm formation on different gentamicin-loaded polymethylmethacrylate bone cements. Biomaterials 22, 1607-1611 (2001)
7. Marra F., Robins G.M., Masri B.A., Duncan C., Wasan K. M., Kwong E.H., Jewesson P.J. Amphotericin B-loaded bone cement to treat osteomyelitis caused by Candida albicans. Canad. J. Surg. 44, 383-386 (2001)
8. Anagnostakos K., Kelm J., Schmitt E., Jung J. Fungal periprosthetic hip and knee joint infections. Clinical experience with a 2-stage treatment protocol. J. Arthoplasty 27, 293-298 (2012)
9. Zasloff M. Antimicrobial peptides of multicellular organisms. Nature 415, 389-395 (2002)
10. Hancock R.E.W., Sahl H.-G. Antimicrobial and host-defense peptides as new anti-infective therapeutic strategies. Natur. Biotech. 24, 1551-1557 (2006)
11. Toke O. Antimicrobial peptides: New candidates in the fight against bacterial infection. Biopolymers (Peptide Science) 80, 717-735 (2005)
12. Giuliani A., Pirri G., Nicoletto S.F. Antimicrobial peptides: An overview of a promising class of therapeutics. Centr. Eur. J. Biol. 2, 1-33 (2007)
13. Zaiou M. Multifunctional antimicrobial peptides: therapeutic targets in several human diseases. J. Mol. Med. 85, 317-329 (2007)
14. Oyston P.C.F, Fox M.A., Richards S.J., Clark G.C. Novel peptide therapeutics for treatment of infections. J. Med. Microb. 58, 977-987 (2009)
15. Baltzer S.A, Brown M.H. Antimicrobial peptides - promising alternatives to conventional antibiotics. J. Mol. Microbiol. Biotechnol. 20, 228-235 (2011)
16. Yeung A.T.Y., Gellatly S.L., Hancock R.E.W. Multifunctional cationic host defence peptides and their clinical applications. Cell. Mol. Life. Sci. 68, 2161-2176 (2011)
17. Otvos L. Jr. Antimicrobial peptides isolated from insects. J. Peptide Sci. 6, 497-511 (2000)
18. Čeřovský V. Antimikrobiální peptidy izolované z hmyzu. Chem. Listy 108, 344-353 (2014)
19. Oren Z., Shai Y. Mode of action of linear amphipathic α-helical antimicrobial peptides. Biopolymers 47, 451-463 (1998)
20. Yeaman M.R., Yount N.Y. Mechanisms of antimicrobial peptide action and resistance. Pharm. Rev. 55, 27-55 (2003)
21. Nguyen L.T., Haney E.F., Vogel H.J. The expanding scope of antimicrobial peptide structures and their modes of action. Trends Biotech. 29, 464-471 (2011)
22. Monincová L., Buděšínský M., Slaninová J., Hovorka O., Cvačka J., Voburka Z., Fučík V., Borovičková L., Bednárová L., Straka J., Čeřovský V. Novel antimicrobial peptides from the venom of the eusocial bee Halictus sexcinctus (Hymenoptera: Halictidae) and their analogs. Amino Acids 39, 763-775 (2010)
23. Fields G.B, Noble R.L. Solid phase peptide synthesis utilizing 9-fluorenylmethoxycarbonyl amino acid. Int. J. Pept. Protein Res. 35, 161-214 (1990)

## Claims

1. Synthetic antimicrobial peptides showing an antimicrobial effect in bone tissue, selected from the group consisting of:
H-Lys-Trp-Met-Lys-Leu-Leu-Lys-Lys-Ile-Leu-Lys-NH₂ (I),
*H-Lys-Trp-Met-Lys-Leu-Leu-Lys-Lys-Ile-Leu-Lys*-NH₂ (II),
H-Gly-Lys-Trp-Met-Lys-Leu-Leu-Lys-Lys-Ile-Leu-Lys-NH₂ (III),
H-Gly*-Lys-Trp-Met-Lys-Leu-Leu-Lys-Lys-Ile-Leu-Lys*-NH₂ (IV),
H-βAla-Lys-Trp-Met-Lys-Leu-Leu-Lys-Lys-Ile-Leu-Lys-NH₂ (V),
H-βAla-*Lys-Trp-Met-Lys-Leu-Leu-Lys-Lys-Ile-Leu-Lys*-NH₂ (VI),
H-Gly-Lys-Trp-Leu-Lys-Leu-Leu-Lys-Lys-Ile-Leu-Lys-NH₂ (VII),
*H-Gly-Lys-Trp-Leu-Lys-Leu-Leu-Lys-Lys-Ile-Leu-Lys*-NH₂ (VIIa)
H-Gly-Lys-Trp-Val-Lys-Leu-Leu-Lys-Lys-Ile-Leu-Lys-NH₂ (VIII),
H-Gly*-Lys-Trp-Val-Lys-Leu-Leu-Lys-Lys-Ile-Leu-Lys*-NH₂ (IX)
H-Gly-Lys-Trp-Lys-Lys-Leu-Leu-Lys-Lys-Ile-Leu-Lys-NH₂ (X),
*H-Gly-Lys-Trp-Lys-Lys-Leu-Leu-Lys-Lys-Ile-Leu-Lys*-NH₂ (Xa),
H-Gly-Lys-Trp-Met-Lys-Met-Leu-Lys-Lys-Ile-Leu-Lys-NH₂ (XI),
*H-Gly-Lys-Trp-Met-Lys-Met-Leu-Lys-Lys-Ile-Leu-Lys-*NH₂ (XIa),
H-Gly-Lys-Trp-Met(O)-Lys-Leu-Leu-Lys-Lys-Ile-Leu-Lys-NH₂ (XII),
*H-Gly-Lys-Trp-Met(O)-Lys-Leu-Leu-Lys-Lys-Ile-Leu-Lys*-NH₂ (XIIa),
H-Gly-Lys-Trp-Met-Lys-Leu-*Leu*-Lys-Lys-Ile-Leu-Lys-NH₂ (XIII)
*H-Gly-Lys-Trp-Met-Lys-Leu-*Leu*-Lys-Lys-Ile-Leu-Lys-*NH₂ (XIIIa)
wherein the amino acids in D configuration are indicated in italics, L-configuration amino acids are indicated in normal type font,
and wherein H indicates a free N-terminal amino group, -NH₂ indicates an amidated C-terminal carboxyl group of peptides, and Met(O) is methionine sulfoxide.

2. Synthetic peptides according to Claim 1 for use in a method of treatment of infections of musculoskeletal system in orthopaedics and traumatology, particularly for the treatment of infectious diseases of bones or surrounding infected tissue and/or to prevent or eliminate the infectious agent from joint and bone replacements and sealants; and for prevention of infection of orthopaedic implants.

3. Use of synthetic peptides according to Claim 1 for manufacture of medicaments for treatment or prevention of infectious diseases of bones or infected surrounding tissues and/or prevention or elimination of the infectious agents from joint and bone replacements and sealants, and for the prevention of infection of orthopaedic implants.

4. Synthetic peptides according to Claim 1 for use in a mixture with a carrier selected from the group consisting of calcium phosphate, calcium sulphate, bioactive glass, apatite-wollastonite ceramic bioglass, hydrogel, collagen, bone grafts, bone cement, synthetic polymers selected from polymethyl methacrylate, copolymers of methyl methacrylate and hydroxyethyl methacrylate, polyanhydride, polylactide, polyglycolide, copolymers of hydroxybutyrate and hydroxyvalerate, polyhydroxyalkanoate, polycaprolactone, gelatine sponges with glycerol, composites composed of polymers and/or mineral carriers, in a method of treatment of infectious bone diseases or of infected surrounding tissues and/or for the prevention or elimination of infectious agents from joint and bone replacements and sealants, and for prevention of infection of orthopaedic implants.

5. A pharmaceutical composition **characterized in that** it comprises a therapeutically effective amount of at least one peptide from the group of peptides of formulae according to Claim 1, optionally a second active ingredient, which is an antibiotic or antifungal agent, and at least one pharmaceutically acceptable carrier, filler and/or diluent.

6. The pharmaceutical composition according to Claim 5 for use in the treatment and/or prevention of infectious diseases of bones or infected surrounding tissues and/or for prevention or elimination of the infectious agent from joint and bone replacements and sealants, and for the prevention of infectious complications after implantation of joint replacements and after osteosynthesis.

7. A composition for medical use, comprising synthetic peptides according to Claim 1 and a carrier selected from the group consisting of calcium phosphate, calcium sulphate, bioactive glass, apatite-wollastonite ceramic bioglass, hydrogel, collagen, bone grafts, bone cement, synthetic polymers selected from polymethyl methacrylate, copolymers of methyl methacrylate and hydroxyethyl methacrylate, polyanhydride, polylactide, polyglycolide, copolymers of hydroxybutyrate and hydroxyvalerate, polyhydroxyalkanoate, polycaprolactone, gelatine sponges with glycerol, composites composed of polymers and/or mineral carriers

## Patentansprüche

1. Synthetische antimikrobielle Peptide, die eine antimikrobielle Wirkung im Knochengewebe zeigen, ausgewählt aus der Gruppe bestehend aus:
H-Lys-Trp-Met-Lys-Leu-Leu-Lys-Lys-Ile-Leu-Lys-NH₂ (I),
*H-Lys-Trp-Met-Lys-Leu-Leu-Lys-Lys-Ile-Leu-Lys*-NH₂ (II),
H-Gly-Lys-Trp-Met-Lys-Leu-Leu-Lys-Lys-Ile-Leu-Lys-NH₂ (III),
H-Gly*-Lys-Trp-Met-Lys-Leu-Leu-Lys-Lys-Ile-Leu-Lys*-NH₂ (IV),
H-βAla-Lys-Trp-Met-Lys-Leu-Leu-Lys-Lys-Ile-Leu-Lys-NH₂ (V),
H*-*βAla-*Lys-Trp-Met-Lys-Leu-Leu-Lys-Lys-Ile-Leu-Lys*-NH₂ (VI),
H-Gly-Lys-Trp-Leu-Lys-Leu-Leu-Lys-Lys-Ile-Leu-Lys-NH₂ (VII),
*H-Gly-Lys-Trp-Leu-Lys-Leu-Leu-Lys-Lys-Ile-Leu-Lys-*NH₂ (VIIa)
H-Gly-Lys-Trp-Val-Lys-Leu-Leu-Lys-Lys-Ile-Leu-Lys-NH₂ (VIII),
H-Gly*-Lys-Trp-Val-Lys-Leu-Leu-Lys-Lys-Ile-Leu-Lys*-NH₂ (IX)
H-Gly-Lys-Trp-Lys-Lys-Leu-Leu-Lys-Lys-Ile-Leu-Lys-NH2 (X),
*H-Gly-Lys-Trp-Lys-Lys-Leu-Leu-Lys-Lys-Ile-Leu-Lys*-NH₂ (Xa),
H-Gly-Lys-Trp-Met-Lys-Met-Leu-Lys-Lys-Ile-Leu-Lys-NH₂ (XI),
*H-Gly-Lys-Trp-Met-Lys-Met-Leu-Lys-Lys-Ile-Leu-Lys*-NH₂ (XIa),
H-Gly-Lys-Trp-Met(O)-Lys-Leu-Leu-Lys-Lys-Ile-Leu-Lys-NH₂ (XII),
*H-Gly-Lys-Trp-Met(O)-Lys-Leu-Leu-Lys-Lys-Ile-Leu-Lys*-NH₂ (XIIa),
H-Gly-Lys-Trp-Met-Lys-Leu-*Leu*-Lys-Lys-Ile-Leu-Lys-NH₂ (XIII)
*H-Gly-Lys-Trp-Met-Lys-Leu-*Leu*-Lys-Lys-Ile-Leu-Lys*-NH₂ (XIIIa)
wobei die Aminosäuren in D-Konfiguration *kursiv* angegeben sind, L-Konfigurations-Aminosäuren in normaler Schrift angegeben sind,
und wobei H eine freie N-terminale Aminogruppe anzeigt, -NH₂ eine amidierte C-terminale Carboxylgruppe von Peptiden anzeigt und Met(O) Methioninsulfoxid ist.

2. Synthetische Peptide nach Patentanspruch 1 zur Verwendung in einem Verfahren zur Behandlung von Infektionen des Bewegungsapparates in der Orthopädie und Traumatologie, insbesondere zur Behandlung von Infektionskrankheiten der Knochen oder des umgebenden infizierten Gewebes und/oder zur Verhinderung oder Beseitigung des Infektionserregers aus Gelenk- und Knochenersatz und Dichtstoffen; und zur Verhinderung der Infektion von orthopädischen Implantaten.

3. Verwendung von synthetischen Peptiden nach Patentanspruch 1 zur Herstellung von Arzneimitteln zur Behandlung oder Prävention von Infektionskrankheiten von Knochen oder infizierten umgebenden Geweben und/oder zur Vorbeugung oder Beseitigung der Infektionserreger aus Gelenk- und Knochenersatz und Versiegelungsmitteln sowie zur Vorbeugung einer Infektion von Orthopäden Implantate.

4. Synthetische Peptide nach Patentanspruch 1 zur Verwendung in einer Mischung mit einem Träger ausgewählt aus der Gruppe bestehend aus Kalziumphosphat, Kalziumsulfat, bioaktivem Glas, Apatit-Wollastonit-Keramik-Bioglas, Hydrogel, Kollagen, Knochentransplantaten, Knochenzement, synthetischen Polymeren ausgewählten aus Polymethyl Methacrylat, Co-Polymere von Methyl Methacrylat und Hydroxyethyl Methacrylat, Polyanhydrid, Polylactid, Polyglycolid, Co-Polymere von Hydroxybutyrat und Hydroxyvalerat, Polyhydroxyalkanoat, Polycaprolacton, Gelatineschwämme mit Glycerin, Verbundwerkstoffe, die aus Polymeren und/oder Mineralträger bestehen, zur Behandlung von infektiösen Knochenerkrankungen oder infizierten umgebenden Geweben und/oder zur Verhinderung oder Beseitigung von Infektionserregern aus Gelenk- und Knochenersatz und Versiegelungsmitteln und zur Verhinderung der Infektion von orthopädischen Implantaten.

5. Eine pharmazeutische Zusammensetzung, die **dadurch gekennzeichnet ist, dass** sie eine therapeutisch wirksame Menge von mindestens einem Peptid aus der Gruppe von Peptiden der Formel nach Patentanspruch 1, gegebenenfalls einen zweiten Wirkstoff, der ein Antibiotikum oder ein Antimykotikum ist, und mindestens einen pharmazeutisch verträglichen Träger, Füllstoff und/oder Verdünnungsmittel umfasst.

6. Die pharmazeutische Zusammensetzung nach Patentanspruch 5 zur Verwendung bei der Behandlung und/oder Prävention von Infektionskrankheiten von Knochen oder infizierten umgebenden Geweben und/oder zur Prävention oder Eliminierung des Infektionserregers aus Gelenk- und Knochenersatz und Versiegelungsmitteln und zur Prävention von infektiösen Komplikationen nach Implantation von Gelenkersatz und nach Osteosynthese.

7. Eine Zusammensetzung zur medizinischen Verwendung, die synthetische Peptide nach Patentanspruch 1 und einen Träger ausgewählt aus der Gruppe bestehend aus Kalziumphosphat, Kalziumsulfat, bioaktivem Glas, Apatit-Wollastonit-Keramik-Bioglas, Hydrogel, Kollagen, Knochentransplantaten, Knochenzement, synthetischen Polymeren ausgewählten aus Polymethyl Methacrylat, Co-Polymeren von Methyl Methacrylat und Hydroxyethyl Methacrylat, Polyanhydrid, Polylactid, Polyglycolid, Co-Polymeren von Hydroxybutyrat und Hydroxyvalerat, Polyhydroxyalkanoat, Polycaprolacton, Gelatineschwämmen mit Glycerin, Verbundstoffen aus Polymeren und/oder Mineralstoffen.

## Revendications

1. Peptides antimicrobiens synthétiques présentant un effet antimicrobien dans le tissu osseux, sélectionnés dans le groupe comprenant:
H-Lys-Trp-Met-Lys-Leu-Leu-Lys-Lys-Ile-Leu-Lys-NH₂ (I),
*H-Lys-Trp-Met-Lys-Leu-Leu-Lys-Lys-Ile-Leu-Lys*-NH₂ (II),
H-Gly-Lys-Trp-Met-Lys-Leu-Leu-Lys-Lys-Ile-Leu-Lys-NH₂ (III),
H-Gly*-Lys-Trp-Met-Lys-Leu-Leu-Lys-Lys-Ile-Leu-Lys-*NH₂ (IV),
H-βAla-Lys-Trp-Met-Lys-Leu-Leu-Lys-Lys-Ile-Leu-Lys-NH₂ (V),
H-βAla*-Lys-Trp-Met-Lys-Leu-Leu-Lys-Lys-Ile-Leu-Lys*-NH₂ (VI),
H-Gly-Lys-Trp-Leu-Lys-Leu-Leu-Lys-Lys-Ile-Leu-Lys-NH₂ (VII),
*H-Gly-Lys-Trp-Leu-Lys-Leu-Leu-Lys-Lys-Ile-Leu-Lys*-NH₂ (VIIa)
H-Gly-Lys-Trp-Val-Lys-Leu-Leu-Lys-Lys-Ile-Leu-Lys-NH₂ (VIII),
H-Gly-*Lys-Trp-Val-Lys-Leu-Leu-Lys-Lys-Ile-Leu-Lys*-NH₂ (IX)
H-Gly-Lys-Trp-Lys-Lys-Leu-Leu-Lys-Lys-Ile-Leu-Lys-NH₂ (X),
*H-Gly-Lys-Trp-Lys-Lys-Leu-Leu-Lys-Lys-Ile-Leu-Lys*-NH₂ (Xa),
H-Gly-Lys-Trp-Met-Lys-Met-Leu-Lys-Lys-Ile-Leu-Lys-NH₂ (XI),
*H-Gly-Lys-Trp-Met-Lys-Met-Leu-Lys-Lys-Ile-Leu-Lys*-NH₂ (XIa),
H-Gly-Lys-Trp-Met(O)-Lys-Leu-Leu-Lys-Lys-Ile-Leu-Lys-NH₂ (XII),
*H-Gly-Lys-Trp-Met(O)-Lys-Leu-Leu-Lys-Lys-Ile-Leu-Lys*-NH₂ (XIIa),
H-Gly-Lys-Trp-Met-Lys-Leu-*Leu*-Lys-Lys-Ile-Leu-Lys-NH₂ (XIII)
*H-Gly-Lys-Trp-Met-Lys-Leu-*Leu*-Lys-Lys-Ile-Leu-Lys*-NH₂ (XIIIa)
où les acides aminés en configuration D sont indiqués en italique, les acides aminés en configuration L sont indiqués en caractères de type normal,
et où H indique un groupe amino N-terminal libre, -NH₂ indique un groupe carboxyle C-terminal amidé de peptides, et Met(O) est le sulfoxyde de méthionine.

2. Peptides synthétiques selon la revendication 1 pour utilisation dans une méthode de traitement des infections du système musculo-squelettique en orthopédie et en traumatologie, en particulier pour le traitement des maladies infectieuses des os ou des tissus infectés environnants et/ou pour prévenir ou éliminer l'agent infectieux des remplacements articulaires et osseux et des scellants; et pour la prévention de l'infection des implants orthopédiques.

3. Utilisation de peptides synthétiques selon la revendication 1 pour la fabrication de médicaments pour le traitement ou la prévention des maladies infectieuses des os ou des tissus environnants infectés et/ou la prévention ou l'élimination des agents infectieux des remplacements articulaires et osseux et des scellants, et pour la prévention des infection d'implants orthopédiques.

4. Peptides synthétiques selon la revendication 1 pour utilisation dans un mélange avec un support choisi dans le groupe constitué par le phosphate de calcium, le sulfate de calcium, le verre bioactif, le bioglasse céramique d'apatite-wollastonite, l'hydrogel, le collagène, les greffes osseuses, le ciment osseux, les polymères synthétiques sélectionnés à partir de polyméthacrylate de méthyle, de copolymères de méthacrylate de méthyle et de méthacrylate d'hydroxyéthyle, de polyanhydride, de polylactide, de polyglycolide, de copolymères d'hydroxybutyrate et d'hydroxyvalérate, polyhydroxyalcanoate, polycaprolactone, éponges de gélatine avec glycérol, composites composés de polymères et/ou de supports minéraux, dans une méthode de traitement des maladies osseuses infectieuses ou des tissus environnants infectés et/ou pour la prévention ou l'élimination des agents infectieux des remplacements articulaires et osseux et scellants, et pour la prévention de l'infection des implants orthopédiques.

5. Composition pharmaceutique **caractérisée en ce qu'**elle comprend une quantité thérapeutiquement efficace d'au moins un peptide du groupe des peptides de formules selon la revendication 1, éventuellement un deuxième principe actif, qui est un antibiotique ou un antifongique, et au moins un support, une charge et/ou un diluant pharmaceutiquement acceptables.

6. Composition pharmaceutique selon la revendication 5, pour utilisation dans le traitement et/ou la prévention des maladies infectieuses des os ou des tissus environnants infectés et/ou pour la prévention ou l'élimination de l'agent infectieux des remplacements articulaires et osseux et scellants, et pour la prévention des complications infectieuses après implantation de prothèses articulaires et après ostéosynthèse.

7. Composition à usage médical, comprenant des peptides synthétiques selon la revendication 1 et un support choisi dans le groupe constitué par le phosphate de calcium, le sulfate de calcium, le verre bioactif, le bioglasse céramique apatite-wollastonite, l'hydrogel, le collagène, les greffes osseuses, le ciment osseux, synthétique polymères choisis parmi le polyméthacrylate de méthyle, les copolymères de méthacrylate de méthyle et de méthacrylate d'hydroxyéthyle, le polyanhydride, le polylactide, le polyglycolide, les copolymères d'hydroxybutyrate et d'hydroxyvalérate, le polyhydroxyalcanoate, la polycaprolactone, les éponges de gélatine avec du glycérol, les composites composés de polymères et/ou de supports minéraux.


### SEQUENCE LISTING

<110> Ustav organicke chemie a biochemie AV CR, v.v.i. Fakultni nemocnice v Motole
<120> Synthetic antimicrobial peptides and their applications for the treatment and prevention of musculoskeletal infections
<130> P1528PC00
<160> 13
<170> PatentIn version 3.5
<210> 1
   <211> 11
   <212> PRT
   <213> Artificial
<220>
   <223> Synthesised using solid phase peptide synthesis.
<400> 1
   Lys Trp Met Lys Leu Leu Lys Lys Ile Leu Lys
   1               5                   10
<210> 2
   <211> 11
   <212> PRT
   <213> Artificial
<220>
   <223> Synthesised using solid phase peptide synthesis.
<400> 2
   Lys Trp Met Lys Leu Leu Lys Lys Ile Leu Lys
   1               5                   10
<210> 3
   <211> 12
   <212> PRT
   <213> Artificial
<220>
   <223> Synthesized by the method of solid phase peptide synthesis (SPPS).
<400> 3
   Gly Lys Trp Met Lys Leu Leu Lys Lys Ile Leu Lys
   1               5                   10
<210> 4
   <211> 12
   <212> PRT
   <213> Artificial
<220>
   <223> Synthesized by the method of solid phase peptide synthesis (SPPS).
<400> 4
   Gly Lys Trp Met Lys Leu Leu Lys Lys Ile Leu Lys
   1               5                   10
<210> 5
   <211> 12
   <212> PRT
   <213> Artificial
<220>
   <223> Synthesized by the method of solid phase peptide synthesis (SPPS).
<220>
   <221> X
   <222> (1)..(1)
   <223> bAla
<400> 5
   Xaa Lys Trp Met Lys Leu Leu Lys Lys Ile Leu Lys
   1               5                   10
<210> 6
   <211> 12
   <212> PRT
   <213> Artificial
<220>
   <223> Synthesized by the method of solid phase peptide synthesis (SPPS).
<220>
   <221> X
   <222> (1)..(1)
   <223> bAla
<400> 6
   Xaa Lys Trp Met Lys Leu Leu Lys Lys Ile Leu Lys
   1 5 10
<210> 7
   <211> 12
   <212> PRT
   <213> artificial
<220>
   <223> Synthesized by the method of solid phase peptide synthesis (SPPS).
<400> 7
   Gly Lys Trp Leu Lys Leu Leu Lys Lys Ile Leu Lys
   1 5 10
<210> 8
   <211> 12
   <212> PRT
   <213> Artificial
<220>
   <223> Synthesized by the method of solid phase peptide synthesis (SPPS).
<400> 8
   Gly Lys Trp Val Lys Leu Leu Lys Lys Ile Leu Lys
   1 5 10
<210> 9
   <211> 12
   <212> PRT
   <213> Artificial
<220>
   <223> Synthesized by the method of solid phase peptide synthesis (SPPS).
<400> 9
   Gly Lys Trp Val Lys Leu Leu Lys Lys Ile Leu Lys
   1 5 10
<210> 10
   <211> 12
   <212> PRT
   <213> artificial
<220>
   <223> Synthesized by the method of solid phase peptide synthesis (SPPS).
<400> 10
   Gly Lys Trp Lys Lys Leu Leu Lys Lys Ile Leu Lys
   1 5 10
<210> 11
   <211> 12
   <212> PRT
   <213> artificial
<220>
   <223> Synthesized by the method of solid phase peptide synthesis (SPPS).
<400> 11
   Gly Lys Trp Met Lys Met Leu Lys Lys Ile Leu Lys
   1 5 10
<210> 12
   <211> 12
   <212> PRT
   <213> artificial
<220>
   <223> Synthesized by the method of solid phase peptide synthesis (SPPS).
<220>
   <221> X
   <222> (4)..(4)
   <223> methionine sulfoxide
<400> 12
   Gly Lys Trp Xaa Lys Leu Leu Lys Lys Ile Leu Lys
   1 5 10
<210> 13
   <211> 12
   <212> PRT
   <213> artificial
<220>
   <223> Synthesized by the method of solid phase peptide synthesis (SPPS).
<400> 13
   Gly Lys Trp Met Lys Leu Leu Lys Lys Ile Leu Lys
   1 5 10